# EUROPEAN PATENT SPECIFICATION

(11) **EP 2 246 017 B1**
(45) Date of publication and mention of the grant of the patent: **27.11.2019**
(21) Application number: 09159194.1
(22) Date of filing: 30.04.2009
(51) Int. Cl.: A61F 13/15

(54) **Absorbent article**
Saugfähiger Artikel
Article absorbant

(43) Date of publication of application: 03.11.2010
(73) Proprietor: Attends Healthcare AB, 578 24 Aneby (SE)
(72) Inventor: Svensson, Ida, 554 50, Jönköping (SE); Persson, Christer, 578 31, Aneby (SE); Lindqvist, Arne, 152 57, Södertälje (SE); Håkansson, Mikael, 573 37, Tranås (SE)
(74) Representative: AWA Sweden AB

(56) References cited:
- EP-A1- 0 617 602
- EP-A1- 1 688 111
- EP-A2- 0 613 671
- US-A- 6 020 536
- US-A1- 2002 169 428

## Description

### Technical field

The present disclosure relates to an absorbent article, such as an incontinence pad which is arranged to be worn in an undergarment of a user. The absorbent article comprises a top sheet, a back sheet, and arranged in between the top sheet and the back sheet, an acquisition layer and two absorbent cores.

### Background of the invention

A wide variety of absorbent articles for collecting body fluids are known in the art. Absorbent articles in general, and incontinence pads in particular usually comprise a central absorbent core disposed between a topsheet, defining a surface for facing the body of a user, and a backsheet, defining a surface for facing away from the user's body.

Incontinence pads intended to be worn by adult users generally need to have a high absorbent capacity. It is also desirable that the incontinence pads are comfortable and discrete. In order to achieve discrete absorbent articles with a high absorbent capacity, the absorbent cores of the articles are preferably thin, and comprise super absorbent polymers (SAP).

US 2002/169428 A1 discloses an absorbent article having an absorbent core comprising first and second absorbent layers.

EP 1 688 111 A1 discloses an incontinence pad which is thin and has a high absorbent capacity. The incontinence pad has two absorbent cores and the area of the uppermost of the absorbent cores is smaller than the area of the lowermost absorbent core. The relative positions and areas of the absorbent cores are chosen in order to optimize the absorbent capacity of the incontinence pad at the same time as the material needed to manufacture the incontinence pad is minimized.

However, there remains a need for discrete incontinence pads, having a high absorbent capacity, which incontinence pads furthermore provide an even more reduced risk for leakage, and which incontinence pads provide even better fitting properties and are even more comfortable to the wearer.

### Summary

It is an object of the present invention to provide an improved solution that alleviates the mentioned drawbacks with present devices. According to an aspect of the solution, this object is achieved by an absorbent article according to claim 1. The absorbent article comprises a substantially fluid impervious backsheet, a substantially fluid permeable topsheet, an acquisition layer for collecting and distributing fluid, a top core for absorbing fluid, a bottom core for absorbing fluid, elastic members. The acquisition layer, the top core and the bottom core are arranged between the topsheet and the backsheet. The acquisition layer is arranged between the topsheet and the top core, and the bottom core is arranged between the backsheet and the top core. The absorbent article extends along a longitudinal axis from its front end towards its rear end. It has longitudinal side edges extending along the longitudinal axis. The elastic members are arranged adjacent to at least a portion of each longitudinal side edge and each elastic member has a tension in a direction along the longitudinal side edge. The top and the bottom cores of the absorbent article comprise absorbent material that is compressed to a total density for the top and bottom core of at least 0,13 g/cm³, preferably at least 0,15 g/cm³, most preferably at least 0,18 g/cm³.

Thereby, an absorbent article is achieved, which absorbent article will fit naturally to the body of the wearer during use, since the absorbent article will spontaneously take the form of a cup. The cup shape results in reducing the leakage risk, and makes the absorbent article comfortable. The relatively high density of the absorbent cores and the characteristics of the elastic members facilitate the spontaneous cup shaping.

According to an embodiment of the invention, the longitudinal extent of the top core is 60-90 % of the longitudinal extent of the bottom core, preferably 62-68 %, more preferably 64-68, most preferably 65-67 %.

Thereby, an absorbent article is achieved, for which there are high absorbent regions comprising both a top core and a bottom core, and other, thinner regions where only a bottom core is present. In that way a high absorption capacity is achieved in regions of the absorbent article where it is mostly needed, whereas other regions of the absorbent article can be thinner, and thereby easily configurable into a suitable shape.

According to another embodiment of the invention, an area of a body-facing surface of the top core is 50-62 % of an area of a body-facing surface of the bottom core, preferably 52-61 %, more preferably 54-61 %, most preferably 55-60 %. Thereby, an absorbent article is achieved, for which there are high absorbent regions comprising both a top core and a bottom core, and other, thinner regions where only a bottom core is present. In that way a high absorption capacity is achieved in regions of the absorbent article where it is mostly needed, whereas other regions of the absorbent article can be thinner, and thereby easily configurable into a suitable shape.

According to yet another embodiment of the invention, the elastic members are foam elastic members. Foam elastic members provide a soft feeling for the user, and are comfortable.

According to other embodiments of the invention, the elastic members comprise elastic threads or an elastic film material.

According to yet another embodiment of the invention each elastic member is arranged to adopt an initial relaxed length, when no external forces are acting on the elastic member, and an outstretched length, when it is attached to the absorbent article and the absorbent article is in a flat outstretched condition within a plane, the outstretched length being 140-180% of the initial relaxed length, preferably 150-170%, most preferably 155-165%.

Thereby, a suitable elastic member tension is achieved when the absorbent article is in a use condition, facilitating the spontaneous cup shaping of the absorbent article.

According to yet another embodiment of the invention, each elastic member is arranged such that it has an outstretched length when it is attached to the absorbent article and the absorbent article is in a flat outstretched condition, within a plane, which outstretched length is about 40-61 % of the length of the bottom core 70, preferably 45-56 % most preferably 48-53 %.

Such a length ratio between the elastic members and the bottom core will also facilitate the spontaneous cup shaping of the absorbent article.

According to yet another embodiment of the invention, the top core and the bottom core are hour glass shaped. This shape is particularly suitable as it is well suited to fit between the wearer's legs during use of the absorbent article at the same time as an adequate absorbent capacity is provided.

According to yet another embodiment of the invention, the top core and the part of the bottom core which is covered by the top core, have a retention value that corresponds to 60-90 %, preferably 70-80 % of a total retention value of the top core and the bottom core.

This means that the region of the absorbent article in which both a top core and a bottom core is present will have a high absorption and retention capacity, such that this region is capable of absorbing a major part of the liquid which reaches the absorbent cores.

According to another embodiment of the invention, the longitudinal extent of the bottom core is 200-550 mm, preferably 350-500 mm, most preferably 445-460 mm. An absorbent article according to the invention, for which the bottom core has a longitudinal extent like that, will have a relatively high absorbent capacity.

According to another embodiment of the invention a distance in the transversal direction between an elastic member and the bottom core, in a position along the longitudinal direction where the bottom core has its smallest lateral extent, is 4-12 mm, preferably 6-11 mm, most preferably 9-10 mm. Thereby, the side margins of the absorbent article, which side margins comprise the elastic members, will more easily get an angle to the body-facing surface of the absorbent article, and the side margins will rise above the body-facing surface of the absorbent article. In that way, the leakage risk is reduced.

A distance in the transversal direction between a side edge of the absorbent article and a proximal side edge of the bottom core, in a position along the longitudinal direction where the bottom core has its smallest lateral extent, is 15-25 mm, preferably 18-24 mm, most preferably 20-23 mm. Thereby, the side margins of the absorbent article, which side margins comprise the elastic members, will more easily get an angle to the body-facing surface of the absorbent article, and the side margins will rise above the body-facing surface of the absorbent article. In that way, the leakage risk is reduced.

According to another embodiment of the invention, the total thickness of the absorbent cores is less than 7 mm, preferably less than 6 mm. Thereby, an absorbent article is provided which is discrete. The thinness of the absorbent core will also facilitate the spontaneous cup shaping of the absorbent article.

According to another embodiment of the invention, the bottom core has an embossed pattern. In the embossed pattern, natural folding lines will appear, which makes the bottom core flexible. Moreover, the embossed pattern enhances the wicking capacity of the capillaries in the bottom core.

### Brief description of the drawings

The invention will be understood and appreciated more fully from the following detailed description, taken in conjunction with the drawings, in which:
Fig. 1 is a side elevational view of an exemplary, non-limiting embodiment of an absorbent article of the present invention.
Fig. 2 and fig. 3 are top plan views of an absorbent article according to an embodiment of the present invention its flat-out, uncontracted state (i.e. with all elastic induced gathering and contraction removed), and with the topsheet removed.
Fig. 4 is an exploded view of an embodiment of the absorbent article.
Fig. 5a shows a partial cross section of one preferred embodiment of the absorbent article which cross section is taken along line I-I in Fig. 2.
Fig. 5b shows a partial cross section of one alternative preferred embodiment of the absorbent article, which cross section is also taken along line I-I in Fig.2.
Fig. 6 is a side elevational view, showing the outer shape of a cross section of an absorbent article according to an embodiment of the invention, which cross section is taken along line I-I in Fig. 2.

### Description of embodiments

The present invention will be described more fully hereinafter with reference to the accompanying drawings, in which preferred embodiments of the invention are shown. The invention may, however, be embodied in many different forms and should not be construed as limited to the embodiments set forth herein; rather these embodiments are provided so that this disclosure will be thorough and complete, and will fully convey the scope of the invention to those skilled in the art. In the drawings, like numbers refer to like elements.

As used herein, the following terms have the following meanings:
"Absorbent articles" refers to devices that absorb and contain liquid, and more specifically refers to incontinence pads, arranged to absorb urine and to be placed in an undergarment of an adult wearer.
"Longitudinal" is a direction running parallel to the maximum linear dimension of the article. The longitudinal direction is parallel to the y-axis in the drawings.

If not otherwise stated, the term "length" is used for an extent along the longitudinal direction of the absorbent article.

The "lateral" or "transverse" direction is orthogonal to the longitudinal direction. The lateral direction is consequently parallel to the x-axis in the drawings.

If not otherwise stated, the term "width" is used for an extent along the transversal direction of the absorbent article.

The "horizontal plane" refers to the x-y plane in the figures, i.e. a plane that is congruent with the longitudinal and transverse directions of the absorbent article.

As used herein, "up" or "upward" refers to a direction from the backsheet toward the topsheet of the absorbent article shown in the figures, which direction is parallel to the z-axis in the figures. "Down" or "downward" refers to a direction from the topsheet toward the backsheet of the absorbent article shown in the figures. The use of the terms "upper" "lower", "above" and "below" is congruent with the definitions of upward and downward.

If not otherwise stated, the terms "thickness" and "height" are used for extents along the z-direction of the absorbent article.

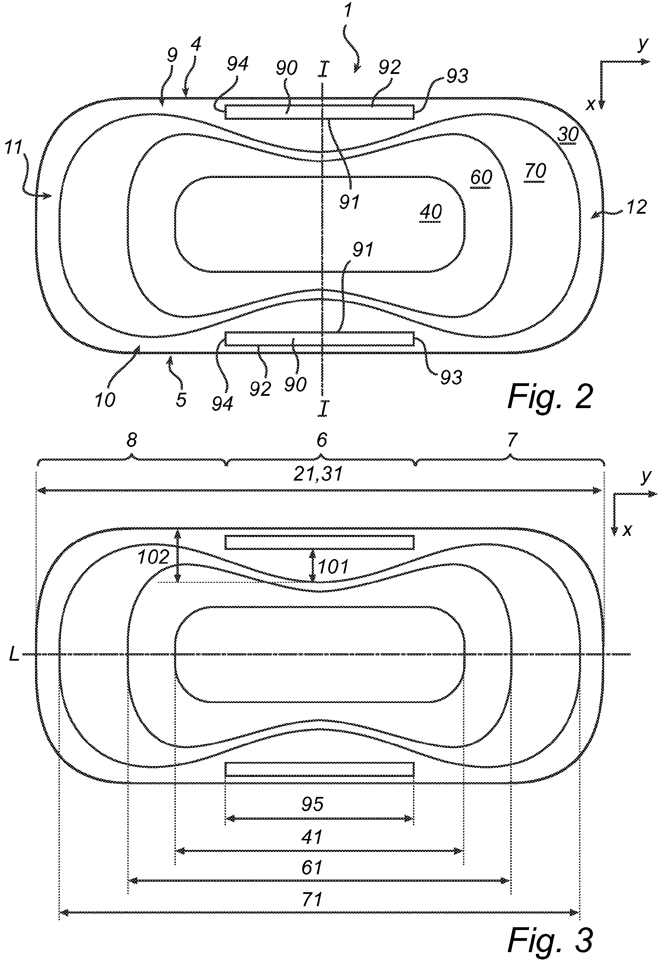

In general, the "forward direction" refers to the positive y-direction in Figs. 2 and 3, whereas the "rearward direction" refers to the negative y-direction in Figs. 2 and 3.

"Disposable," in reference to absorbent articles, means that the absorbent articles are generally not intended to be laundered or otherwise restored or reused as absorbent articles (i.e., they are intended to be discarded after a single use and, preferably, to be recycled, composted or otherwise discarded in an environmentally compatible manner).

As used herein, the term "body-facing surface" generally refers to a surface oriented towards the body when fitted to a wearer.

As used herein, the term "garment-facing surface" generally refers to a surface oriented opposite the body-facing surface when fitted to a wearer.

The terms "permeable" and "impermeable" refer to the penetrability of materials in the context of the intended usage of disposable absorbent articles. Specifically, the term "permeable" refers to a layer or a layered structure having pores or openings that permit liquid to pass through its thickness in the absence of a forcing pressure. Conversely, the term "impermeable" generally refers to articles and/or elements that are not penetrative by fluid in the absence of a forcing pressure (aside from natural forces such as gravity).

"Extendibility" and "extensible" mean that the width or length of the component in a relaxed state can be extended or increased.

"Elastic," "elastomer," and "elastomeric" refer to a material which generally is able to extend to a strain of at least 50% without breaking or rupturing, and is able to recover substantially to its original dimensions after the deforming force has been removed.

"Elastomeric material" is a material exhibiting elastic properties.

"Nonwoven" fabric or web means a web having a structure of individual fibers or threads that are interlaid, but not in a regular or identifiable manner as in a knitted fabric. Nonwoven fabrics or webs have been formed from many processes such as, for example, meltblowing processes, spunbonding processes, air laying processes, and bonded carded web processes.

Unless otherwise noted, "laminated structure" or "laminate" means a structure in which one layer, material, component, web, or substrate is adhesively bonded, at least in part, to another layer, material, component, web, or substrate. A layer, material, component, web, or substrate may be folded over and adhesively bonded to itself to form a "laminated structure" or "laminate."

The term "bonding area" is used for specifying how closely the fibers of a certain material are coupled to each other.

The "retention value" of an object denotes the ability of that object to both absorb and retain liquid.

The term "hour glass shape" refer to an elongate shape wherein a relatively narrower region is present between the relatively wider end regions.

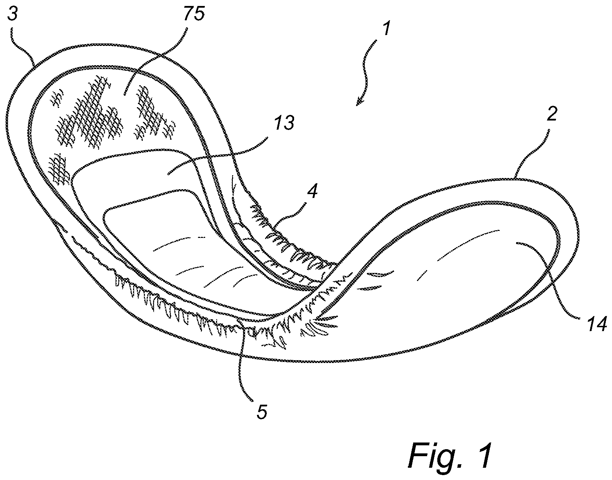

Shown in Figures 1-6 is an absorbent article 1, in accordance with a specific non-limiting example of presentation of the present invention.

With reference to Figs. 1 and 3, an absorbent article 1 according to the present invention has a front end 2, a back end 3, opposing side edges 4, 5, and an longitudinal center line L. The absorbent article 1 has a body-facing surface 13 and a garment-facing surface 14.

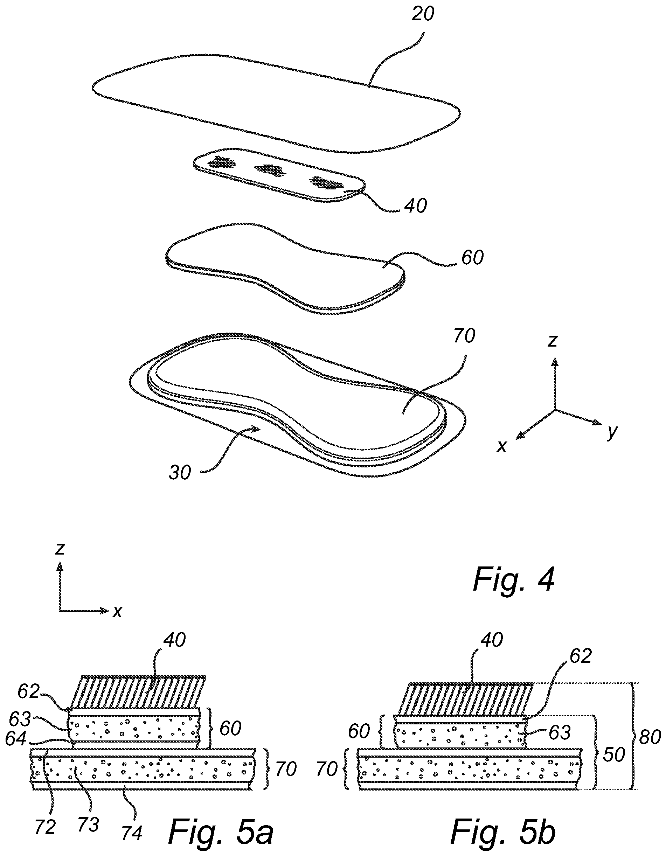

Fig. 4 shows the individual components of an absorbent article according to an embodiment of the present invention. The absorbent article comprises at least four primary components. These include a liquid pervious topsheet 20, a liquid impervious backsheet 30, an acquisition layer 40 and a core component 60, 70. The acquisition layer 40 and the core component 50 are positioned between the topsheet 20 and the backsheet 30. The acquisition layer 40 is positioned between the core component 50 and the topsheet 20.

The core component 50 comprises at least a top core 60 and a bottom core 70. The top core 60 is positioned between the bottom core 70 and the acquisition layer 40. The bottom core 70 is positioned between the top core 60 and the backsheet 30.

The acquisition layer 40 may either be a separate component positioned between the topsheet 20 and the core component 50, or it may be a part of a composite topsheet 20 or a part of the core component 50.

According to a preferred embodiment of the invention, the acquisition layer 40 is a separate component positioned between the topsheet 20 and the core component 50. The core component 50 and the acquisition layer 40 constitute the absorbent structure 80 of the absorbent article 1.

The components of the absorbent article 1 may be comprised of any suitable materials that are capable of being connected in the manner described herein.

### Topsheet

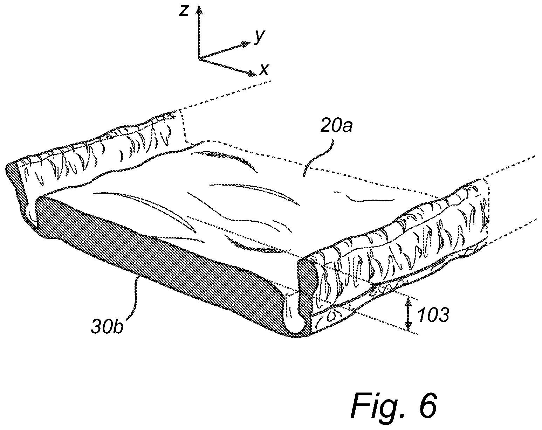

The topsheet 20 is preferably fluid-permeable and has a surface 20a that is intended to face, and abut on, the body of a wearer during use of the absorbent article, see Fig. 6. This surface 20a of the top sheet constitutes the body-facing surface 13 of the absorbent article, as shown in Fig. 1. Since it is fluid-permeable, the topsheet 20 permits liquids to readily penetrate through its thickness. The topsheet 20 is preferably as compliant, soft feeling, and non-irritating to the wearer's skin as possible.

A suitable topsheet 20 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised at least partially of natural fibers (e.g., wood or cotton fibers), synthetic fibers (e.g., polymeric fibers such as polyester, polypropylene, or polyethylene fibers) or from a combination of natural and synthetic fibers.

According to an embodiment of the invention, the topsheet 20 comprises a perforated laminate made of plastic or nonwoven.

According to a preferred embodiment of the invention , the topsheet consists of a nonwoven fabric, made of polypropylene. The bonding area between the fibers in the nonwoven web is preferably 10-22%, most preferably 11% and the basis weight of the nonwoven web may e.g. be 10-20 grams per square meter (gsm), preferably equal to or less than 17 gsm. A topsheet with the above mentioned features will feel dryer to a wearer's skin than a nonwoven topsheet having a higher bonding area, such as 20 %, since the lower bonding area facilitates penetration of liquid into the underlying layers of the absorbent article.

According to another preferred embodiment of the invention, the topsheet is a so called hybrid topsheet, comprising three zones, extending longitudinally and alongside each other. A central zone extends in an area surrounding the longitudinal centre line of the absorbent article. The central zone has longitudinal side edges, and two lateral zones are disposed alongside either of the side edges of the central zone. Each lateral zone has a central longitudinal side edge facing towards the longitudinal centre line L of the absorbent article and a lateral longitudinal side edge, facing away from the longitudinal centre line L of the absorbent article. The central longitudinal side edges of the lateral zones and the side edges of the central zone overlap. The central longitudinal side edges of the lateral zones are attached to the longitudinal side edges of the central zone by means of embossing and/or glueing.

The central zone may e.g. be manufactured from a three-dimensional formed, apertured polypropylene or polyethylene film. Apertured formed films are especially suitable because they are pervious to body exudates and yet non absorbent and have a reduced tendency to allow fluids to pass back through and rewet the wearer's skin. Thus, the surface of the formed film that is in contact with the body remains dry, thereby reducing body soiling, and creating a more comfortable feel for the wearer.

The lateral zones may e.g. be manufactured from a bicomponent non woven web wherein the fibers are bicomponent fibers. Such a non woven web like that is soft feeling, and non-irritating to the wearer's skin.

According to another embodiment the topsheet may comprise only a three-dimensional formed, apertured polypropylene or polyethylene film, which extends over the entire body-facing surface of the absorbent article.

### Backsheet

The backsheet 30 is suitably fluid-impermeable and has a surface 30b that is intended to face away from the body of the wearer during use of the absorbent article. This surface 30b will face the undergarment to which the absorbent article is secured, and constitutes the undergarment-facing surface 14 of the absorbent article 1.

A typical backsheet can be manufactured from a thin plastic film, or another substantially liquid-impermeable material. The backsheet prevents the exudates contained in the absorbent article from wetting articles such as undergarments, which contact the absorbent article.

According to a preferred embodiment of the invention, the backsheet is a breathable textile backsheet, manufactured from laminate of a breathable polyfilm and a nonwoven web. The polyfilm comprises polyethylen and carbon carbonate and is impermeable to fluids but permeable to vapor. The nonwoven web is arranged below the polyfilm and will face the undergarment of the wearer during use of the absorbent article. The nonwoven web provides a soft feeling. Furthermore, the nonwoven web provides a slightly rough surface, which implies that friction appears between the absorbent article and the undergarment in which the article is positioned. The friction assists in keeping the absorbent article in a fixed position in the undergarment.

### Absorbent structure

With reference to Figs. 5a and 5b, an absorbent structure 80 according to the present invention comprises an acquisition layer 40 and a core component 50. The core component 50 comprises a top core 60 and a bottom core 70.

### Acquisition layer

The acquisition layer 40 has two main functions. As a first step, the acquisition layer 40 receives liquids passing through the topsheet and distributes the liquids horizontally within the acquisition layer 40 itself, from the point of initial contact to other parts of the acquisition layer 40. Thereafter, it transfers the liquids to the underlying core component 50.

According to a preferred embodiment of the invention, the absorbent article has an acquisition layer 40 manufactured from cross linked fibers or other types of chemically stiffened fibres. The material is also known as "curly fiber".

The longitudinal extent (length) 41 of the acquisition layer 40 may be shorter than the longitudinal extent of the elements of the core component 50. Hereinafter the lengt 71 of the bottom core 70 will be used as a reference value. The length 41 of the acquisition layer 40 may e.g. be 35-60% of the length 71 of the bottom core 70. According to an embodiment of the invention, the length 41 of the acquisition layer 40 is 43% of the length 71 of the bottom core 70.

The acquisition layer 40 may e.g. have a thickness of 2-3 mm. According to an embodiment of the invention, the thickness of the acquisition layer 40 is 2.2 mm.

The basis weight of the acquisition layer 40 may e.g. be 200-300 grams per square meter (gsm). According to an embodiment of the invention, the basis weight of the acquisition layer 40 is 250 gsm. According to another preferred embodiment of the invention, the acquisition layer 40 is manufactured from a high loft nonwoven web. In that case, the basis weight of the acquisition layer 40 may be 30-80 gsm, preferably 40-60 gsm. The length 41 of an acquisition layer 40 manufactured from a high loft nonwoven web may e.g. be 40-90% of the length 71 of the bottom core 70.

### Core component

A core component according to the present invention comprises a plurality of absorbent cores. In the preferred embodiment shown in Figs. 5a and 5b, the core component 50 comprises two absorbent cores - a top core 60 and a bottom core 70.

As shown in Figs. 2 and 3, the absorbent cores of the core component 50 may be hour-glass shaped. This shape is particularly suitable as it is well suited to fit between the wearer's legs during use of the absorbent article at the same time as an adequate absorbent capacity is provided.

### Top core

The area of the top core in a horizontal plane, is preferably smaller than the area of the bottom core in a horizontal plane. The top core area may e.g. be 40-80 % of the bottom core area, preferably 50-70 %, most preferably 55-65%.

According to an embodiment of the invention, the top core area is about 300 cm² whereas the bottom core area is about 500 cm². Consequently, the top core area is 60 % of the bottom core area in that particular embodiment.

According to the invention, the longitudinal extent (length) 61 of the top core 60 is shorter than the longitudinal extent 71 of the bottom core 70. The top core length 61 may e.g. be 55-69 % of the bottom core length 71, preferably 62-68 %, more preferably 64-68, and most preferably 65-67 %. According to an embodiment of the invention the top core length is 66 % of the bottom core length.

The thickness of the top core may e.g. be 0,5-5 mm, preferably 1-4 mm, most preferably 2-3 mm.

According to an embodiment of the present invention shown in Fig. 5b, the top core comprises two sub layers 62, 63. The uppermost of these two sub layers is a dusting layer 62, consisting of hydrophilic fibers called fluff. The purpose of the dusting layer 62 is primarily to protect the wearer's skin from sharp contents that may be present in the other sub layer 63, of the top core 60. The other sub layer 63 of the top core is hereinafter referred to as the main absorbent layer 63 of the top core 60. Another purpose of the dusting layer is to facilitate the manufacturing process of the absorbent article and to prevent contents in the main absorbent layer 63 from whirling about the machines in which the absorbent articles are assembled.

The basis weight of the dusting layer 62 may e.g. be 30-60 gsm, preferably 30-50 gsm and most preferably 35-45 gsm.

By having such a low value of fluff per area in the dusting layers, a thinner article can be achieved than for an article with a higher value on fluff per area, thereby a more body-shaped article is achieved compared to a product having higher value on fluff per area. Moreover, a more cost-efficient product is achieved.

According to another embodiment of the present invention shown in figure 5a, the top core 60 also comprises a second dusting layer 64, arranged adjacent to a garment-facing surface of the main absorbent layer 63. This second dusting layer may 64 be added in order to ease the manufacturing process of the absorbent article.

The main absorbent layer 63 may be composed of a matrix comprising a mixture of fluff and particles of superabsorbent polymers ("SAPs"). The superabsorbent particles (SAP particles) may be substantially homogeneously mixed with the hydrophilic fluff fibers, or may be non uniformly mixed. For example, the concentrations of superabsorbent particles may be arranged in a non-step-wise gradient through the thickness (Z-direction) of the main absorbent layer 63. The concentrations are preferably lower toward the garment-facing surface of the absorbent article and relatively higher toward the body-facing surface of the absorbent article.

The SAP particles may also be arranged in a generally discrete layer within the matrix of hydrophilic fluff fibers. In addition, two or more different types of SAP particles may be selectively positioned at different locations within or along the fluff fiber matrix.

The amount of SAP in the top core is preferably chosen in order to achieve a suitable absorbent capacity and an appropriate degree of dryness of the absorbent article. Furthermore, the amount of SAP depends on the quality, characteristics and price of the chosen SAP. When the SAP particles have collected liquid they might form SAP blocks if the concentration of SAP is too high. It is desirable to avoid such block formations. Different SAP qualities start forming blocks at different concentrations of SAP.

According to an embodiment of the invention, there is about 5 g SAP and 10 g fluff in the top core 60 (including the main absorbent layer 63 and the dusting layer(s) 62, 64. Those proportions gives a SAP concentration (on weight basis) of 33% (5/(5+10)). According to another embodiment of the invention, there is about 9 g SAP and 15 g fluff in the top core. Those proportions gives a SAP concentration of about 37 % (9/(9+15)).

The material in the top core is preferably compressed such that the density of the top core is at least 0,15 g/cm³, preferably at least 0,17 g/cm³, most preferably at least 0,21 g/cm³.

According to an embodiment of the invention, the density of the top core is 0,21 g/cm³. In that particular case, the density of the SAP present in the core is 0,07 g/cm³ whereas the density of the fluff present in the core is 0,14 g/cm³.

According to another embodiment of the invention, the density of the top core is 0,23 g/cm³. In that particular case, the density of the SAP present in the core is 0,08 g/cm³ whereas the density of the fluff present in the core is 0,15 g/cm³.

During production of absorbent articles according to the present invention, the top core is preferably compressed twice in order to achieve the requested density and thinness.

An absorbent core which is thin and has a density according to the invention will present a high core integrity. An absorbent core having a high core integrity will hold together and keep its original shape also when it is wet, since the SAP and fluff in such an absorbent core will stay in the original positions. An absorbent core with a lower core integrity tend to form lumps of fluff and SAP when it is wet. The presence of such lumps will feel uncomfortable to the wearer of the absorbent article. The lumps might also result in skin irritation, since the absorbent article will get bumpy when it is wet, and thereby, the contact surface between the skin of the wearer and the absorbent article will become irregular and the pressure distribution will become uneven.

### Bottom core

The thickness of the bottom core 70 may e.g. be 0,5-5 mm, preferably 1-4 mm, most preferably 1.8-2.2 mm. According to an embodiment of the invention the thickness of the bottom core is 2 mm.

According to an embodiment of the present invention, the bottom core comprises three sub layers 72, 73, 74. The uppermost and the lowermost of these three sub layers are dusting layers 72, 74, comprising fluff but no SAP. The sub layer 73 which is arranged between the two dusting layers 72, 74 is hereinafter referred to as the main absorbent layer 73 of the bottom core. The purposes of the uppermost dusting layer 72 equal the purposes of the dusting layer 62 adjacent to the body facing surface of the top core 60. The function to protect the wearer's skin from sharp contents that may be present in the main absorbent layer 73 of the bottom core 70 is only needed in areas of the bottom core that are not covered by a top core 60. For manufacturing reasons, the dusting layer 72 is arranged to cover the entire body-facing surface of other sub layer 73. This other sub layer 73 is hereinafter referred to as the main absorbent layer 73 of the bottom core.

The bottom core comprises a second dusting layer 74, arranged adjacent to a garment-facing surface of the main absorbent layer 73. The purpose of this layer is to protect the backsheet and the undergarments of the wearer form sharp contents that may be present in the main absorbent layer 73 of the bottom core.

The basis weight of the bottom core dusting layers 72, 74 may e.g. be 30-75 gsm, preferably 30-50 gsm and most preferably 35-45 gsm.

By having such a low value of fluff per area in the dusting layers, a thinner article can be achieved than for an article with a higher value on fluff per area, thereby a more body-shaped article is achieved compared to a product having higher value on fluff per area. Moreover, a more cost-efficient product is achieved.

According to an embodiment of the invention the basis weight of the first bottom core dusting layer 72 is lower than the basis weight of the second bottom core dusting layer 74. The basis weigh of the second bottom core dusting layer 74 may e.g. be about 75 gsm. The relatively high basis weight is chosen in order to achieve a good protection of the backsheet from sharp contents that may be present in the main absorbent layer 73 of the bottom core 70.

The basis weight of the first bottom core dusting layer 72 may be lower, e.g. 40 gsm, since it is considered important to allow SAP particles to be positioned as close to the body facing surface of the absorbent article as possible. SAP particles close to the body facing surface of the absorbent article will result in a dryer surface adjacent to the skin of the user than would be the case with a relatively thick dusting layer above the SAP.

The main absorbent layer 73 of the bottom core 70 may be composed of a matrix comprising a mixture of fluff and particles of superabsorbent polymers ("SAPs").The superabsorbent particles (SAP particles) may be substantially homogeneously mixed with the hydrophilic fluff fibers, or may be non uniformly mixed. For example, the concentrations of superabsorbent particles may be arranged in a non-step-wise gradient through the thickness (z-direction) of the main absorbent layer 73. The concentrations are preferably lower toward the garment-facing surface of the absorbent article and relatively higher toward the body-facing surface of the absorbent article.The SAP particles may also be arranged in a generally discrete layer within the matrix of hydrophilic fluff fibers. In addition, two or more different types of SAP particles may be selectively positioned at different locations within or along the fluff fiber matrix.

The amount of SAP in the bottom core 70 is preferably chosen in order to achieve a suitable absorbent capacity and an appropriate degree of dryness of the absorbent article. Furthermore, the amount of SAP depends on the quality, characteristics and price of the chosen SAP. When the SAP particles have collected liquid they might form SAP blocks if the concentration of SAP is too high. It is desirable to avoid such block formations. Different SAP qualities start forming blocks at different concentrations of SAP. The amount of SAP may e.g. be 10-50%.

According to an embodiment of the invention, there is 2 g SAP and 17 g fluff in the bottom core. Those proportions gives a SAP concentration (on weight basis) of about 10 % (2/(2+17)).

According to another embodiment of the invention, there is 4 g SAP and 17 g fluff in the top core. Those proportions give a SAP concentration of about 19 %.

According to yet another embodiment of the invention, there is 3 g SAP and 22 g fluff in the top core. Those proportions give a SAP concentration of about 12 %.

The material in the bottom core is preferably compressed such that the density of the bottom core is at least 0,13 g/cm³, preferably at least 0,15 g/cm³, most preferably at least 0,17 g/cm³.According to an embodiment of the invention, the density of the bottom core is 0.17 g/cm³. In that particular case, the density of the SAP present in the core is 0,02 g/cm³ whereas the density of the fluff present in the core is 0,15 g/cm³.

According to another embodiment of the invention, the density of the bottom core is 0.18 g/cm³. In that particular case, the density of the SAP present in the core is 0,03 g/cm³ whereas the density of the fluff present in the core is 0,15 g/cm³.

According to yet another embodiment of the invention, the density of the bottom core is 0.20 g/cm³. In that particular case, the density of the SAP present in the core is 0,06 g/cm³ whereas the density of the fluff present in the core is 0,14 g/cm³.

During production of absorbent articles according to an embodiment of the present invention, the bottom core is compressed three times in order to achieve the requested density and thinness. During the third compression step, an embossed pattern 75 might be added to the bottom core by means of special patterned embossing rolls. The embossing creates a pattern of areas of differing densities. The embossed pattern 75 can be formed in a number of different configurations. It is preferably laid out so as to channel fluids across the body facing surface of the bottom core, in order to enhance absorption.

According to an embodiment of the invention, the embossed pattern 75 comprises diamond-shaped regions separated by embossed lines, having a higher density than the diamond-shaped regions, as shown in Fig. 1.

In addition to their function as channels for leading fluid across the surface of the core, the embossed lines will function as natural folding lines, making the bottom core flexible and pliable. This feature is useful when forming the absorbent article into a cup-shaped configuration, as described more fully hereinafter.

Alternatively, no embossed pattern is added to the bottom core.

### Characteristics of the core component

The material in the bottom core 70 and the top core 60 is preferably compressed such that the total density of the core component 50 is at least 0,13 g/cm³, preferably at least 0,15 g/cm³, most preferably at least 0,17 g/cm³ According to an embodiment of the invention, the total density of the core component is 0.18 g/cm³. According to another embodiment of the invention, the total density of the core component is 0,19 g/cm³, and according to yet another embodiment, the total density is 0.20 g/cm³.

The total thickness of the core component is preferably less than 7 mm, most preferably less than 6 mm. According to an embodiment of the invention, the total thickness of the core component is 5 mm. According to another embodiment, the total thickness of the core component is 4 mm.

### Overall core integrity of core component

The high density of the core component 50 of an absorbent article according to the invention, in combination with the thinness of the core, contribute to an enhanced core integrity, compared to thicker core components having lower densities.Since the bottom core as well as the top core of an absorbent article according to the invention has high core integrities, the overall core integrity of the absorbent article will also be high, resulting in the advantages previously mentioned.

### Wicking capacity of core component

The wicking capacity of the core component 50 is enhanced as a result of the compression performed when manufacturing the absorbent article. The compression makes capillaries present in the core component thin, and these thin capillaries have a better wicking capacity than larger capillaries.

### Retention properties of core component

In an absorbent article according to the invention, the capacity to absorb and retain liquid differs between different regions of the absorbent article. Taken together, the top core and the part of the bottom core which is covered by the top core, have a retention value that corresponds to 60-90 %, preferably 70-80 % of the overall retention value of the core component of the absorbent article. The retetion value indicates how well the absorbent core absorbs liquid and retains it within its structure.

### Leg elastics

To provide improved fit and to help reduce leakage of body exudates from the absorbent article, the side margins of the absorbent article may be elasticized with suitable elastic members such as foam elastic members. As shown in Figs. 1 and 2, the elastic members 90 are preferably disposed adjacent to the periphery of the absorbent article 1, preferably along each side edge 4, 5, so that the elastic members tend to draw and hold the absorbent article 1 against the legs of the wearer. The elastic members might e.g. be generally rectangular, having an inner side edge 91, facing towards the longitudinal centre line L of the absorbent article, and outer side edge 92, facing towards a proximal side edge 4, 5 of the absorbent article 1, a front edge 93 and a rear edge 94, as shown in Fig. 93.

According to an embodiment of the invention the elastic members are foam elastic members manufactured from polyurethane.

The elastic members are secured to the absorbent article 1 in an elastically contractible condition so that in a normally unrestrained configuration, the elastic members effectively contract or gather the absorbent article 1. The elastic members can be secured in an elastically contractible condition in several ways. According to an embodiment of the invention, the elastic members are stretched until their outstretched length is 140-180 %, preferably 150-170 %, most preferably 155-161% of the initial ,unstretched length. Then they are glued to the garment-facing surface of the topsheet while the absorbent article 1 is kept in a flat, un-contracted condition.

When the absorbent article is released, and the elastic members are allowed to revert towards their original shape they will not revert to their initial length, but to a third length, the operative length, which depends on their tension as well as on the force created by the structure to which the elastic members are attached, which force will counteract contraction. In Fig. 1, an absorbent article is shown for which the elastic members 90 have reverted to their operative length. The operative length of an elastic member is longer than the initial length and shorter than the outstretched length 95.

The elastic members may extend along a portion of the length of the absorbent article 1. The length of the elastic members is dictated by the length of the other components of the absorbent article. The other way round, the length of e.g. the top core is also chosen depending on the length of the elastic members. The length 61 of the top core is preferably larger than the outstretched length of the elastic members 90.

The elastic members 90 of the absorbent article are preferably arranged to have a tension such that their outstretched length is 40-61 %, preferably 45-56%, most preferably 48-53% of the length 71 of the bottom core 70.

According to an embodiment of the invention, the outstretched length of the elastic members is 220 mm, when the absorbent article is kept flat, in an outstretched state, and the operative length , which is obtained when the absorbent article is in its natural shape wherein the elastic members are allowed to contract, is 165 mm.

The elastic members might be rectangular, but other shapes are also possible. According to an embodiment of the invention, the elastic members are rectangular, with an initial length of 71 mm and an initial width of 16 mm.

### Side margins and end margins of the absorbent article

The topsheet and the backsheet may have length and width dimensions which are generally larger than, and extend beyond, the corresponding dimensions of the absorbent structure 80, providing for side margins 9, 10 extending past the longitudinal side edges of the absorbent structure 80 and end margins 11, 12 extending past the front edge and back edge of the absorbent structure. The side margins 9, 10 extend along the longitudinal side edges 4, 5 of the absorbent article, whereas the end margins 11, 12 extend along the front end 2 and the back end 3 of the absorbent article.

According to a preferred embodiment of the invention the topsheet is associated with and superimposed on the backsheet. The periphery of the backsheet defines the periphery of the absorbent article. The topsheet is coextensive with the backsheet in the longitudinal direction of the absorbent article, but in the transversal direction there are portions of the topsheet extending past the side edges of the backsheet. These protruding portions are preferably folded around the side edges of the backsheet and the elastic members. The protruding portions may be attached to the outer surface of the backsheet by means of glueing.

Parts of the side margins 9, 10 of an absorbent article according to the invention are shown in Fig. 2.

Since the topsheet and backsheet of the absorbent article have essentially straight side edges without any relatively narrower portions, whereas the absorbent cores might have an hour-glass shape, the width of the side margins will vary alongside the longitudinal extent of the absorbent article. The width 102 of a side margin at a certain point along the longitudinal direction/extent of the absorbent article is hereinafter defined as the distance in the lateral direction between the side edge of the bottom core 70 and the side edge of the absorbent article in that certain point. An example is shown in Fig. 3.

### Cup shape

As shown in Fig. 1, an absorbent article according to the invention will spontaneously take the form of a cup. As a result of this cup shape, the absorbent article will fit naturally to the body of the wearer during use. Moreover, the leakage risk is reduced thanks to the cup shape and the characteristics of the elastic members. Furthermore, the contact surface between the body of the wearer and the absorbent article will be more evenly distributed, resulting in less skin irritation and a more comfortable feeling for the wearer.The relatively high density of the absorbent cores and the characteristics of the elastic members facilitate the spontaneous cup shaping.

According to an embodiment of the present invention, the bottom core is embossed. In the embossed pattern 75 natural folding lines will appear, and consequently, the absorbent article will be easier to form into a cup shape than if the bottom core would not have been embossed. The bottom core will get flexible and pliable thanks to the pattern of "folding lines" created by means of embossing.

Previously known absorbent cores that are arranged to be used for the same purpose as the cores in the absorbent article according to the invention, tend to get creased and crumpled when they are arranged in the crotch region of a user.

The absorbent article has a middle region 6, a front end region 7 and a rear end region 8 The elastic member provides a contractive force which causes the topsheet, the absorbent structure 80 and the backsheet in each of the end regions to extend upwardly, inclined away from the horizontal plane.

The contractive force further causes the elastic members to extend upwardly away from the topsheet on both sides of the middle region of the absorbent article. The elastic members are generally rectangular with opposing side edges, a front edge, and a rear edge. When the absorbent article is held in an uncontracted position, with the elastic members in a stretched-out condition, the elastic members will extend in a plane that is essentially parallel to the horizontal plane. Each elastic member has a lateral side edge facing away from the longitudinal center line of the absorbent article, when the absorbent article is held in an uncontracted position, and a central side edge, facing the longitudinal center line of the absorbent article, when the absorbent article is held in an uncontracted position. When the contractive force is allowed to force the end regions of the absorbent article to extend upwardly, it will also cause the elastic members to extend upwardly away from the top sheet, as shown in Fig. 6.

According to an embodiment of the invention the distance 103 in the z-direction between lateral side edge of each elastic member and the body-facing surface of the topsheet is 14 - 18 mm, in a position where the bottom core has its smallest lateral extent, as seen along the longitudinal direction of the absorbent article.According to another embodiment of the invention, a distance 101 in the x-direction between an inner side edge 91 of the elastic member 90 and a proximal side edge of the bottom core 70, in a position along the longitudinal direction where the bottom core has its smallest lateral extent, is 4-12 mm, preferably 6-11 mm, most preferably 9-10 mm. Such a configuration makes the side margins rise above the body-facing surface of the absorbent article, as best shown in Fig. 6. In that way, the leakage risk is reduced.

A distance 102 in the x-direction between a side edge 4, 5 of the absorbent article 1 and a proximal side edge of the bottom core 70, in a position along the longitudinal direction where the bottom core has its smallest lateral extent, is 15-25 mm, preferably 18-24 mm, most preferably 20-23 mm. As show in the figures, the smallest lateral extent of the absorbent cores 60, 70, is located in the central region 6 of the absorbent article 1, such that the shape of the absorbent cores 60, 70 will provide an absorbent article that fits well in the crotch region of a user.

### Core manufacturing process

In order to achieve a product that spontaneously takes the form of a cup, thin and high density cores are needed. To be able to produce high density cores in a converting machine there are two things of importance: The formation of the core might be very much controlled and made with dispersed and high defibration of the fibers and with a good control of humidity in the formed core.

To achieve the high quality formation in the process with low variations of surface weight (<10%) of the fibers/fluff ,the following process steps are controlled:
Defibration of the fluff sheet is performed efficiently and with a low amount of knots and fines. Typical used is a saw tooth rotor used w/o any screens. The fiber transport to the forming area, might be very short and done with low concentration of fibers in the carrying air. Normal airspeed is about 20 m/s. Unnecessary bends, contractions etc. are avoided in order to avoid turbulence. The core forming might be done without any reduced capacity which means that the flow lines in fiber transport can be "absorbed" by the forming area w/o any redirections of overflowed air. The core forming "screen" is able "to take care" of fibers caring air w/o any high pressure drops. The forming wheel (or similar)and the forming areas might be close to each other to avoid turbulence. The forming hood might be very well aerodynamic adapted to follow the natural way for the flow stream to reduces turbulence.

To achieve the right density in the compression unit in a sustained way, the humidity in the core is controlled. This is made by having good control of humidity in the fluff sheet fed into the mill and a good control of the carrying/process air's humidity. Target is to have a humidity in the core of 8 to 9 %. The humidity of the process air needs to be controlled and adjusted as the environment around the process changes. Normally it's possible to increase the humidity in the core above the feed fluff sheet with a high humidity in the process air.

To get the right density the formed core might be compressed in several steps. The compression also needs a design to keep control of the geometry during compression to avoid changes in dimension of the core by the compression. Several different techniques are available. One option is to build up hydrogen bonding between the fibers. This may need heated compression and special patterned embossing rolls.

### Fastening means

Absorbent articles for wearing in the undergarment of a user generally comprise some kind of fastening means, intended to secure the absorbent article to a body-facing surface of e.g. a crotch portion of the undergarment. The absorbent article is thereby retained in place against the perineal region of the user, as long as he or she wears the undergarment to which the absorbent article is secured.

The fastening means may e.g. be a layer of adhesive material provided on the surface of the absorbent article that faces away from the body of the user, i.e. the undergarment-facing surface.

Prior to use of the absorbent article, the fastening means is preferably covered by a peelable protective layer that prevents soiling of the fastening means and entanglement of the absorbent article when it is wrapped in a package

### Test results

The absorption capacity of absorbent articles according the invention has been tested according to the Post Acquisition Collagen Rewet Method (PACORM) test method, which is described in detail in EP 0 797 967 B1. (In EP 0 797 967 B1 the PACORM test method is referred to as "Acquisition test".)

According to the method, the acquisition speed is defined as the gush volume absorbed (ml) per unit time (s).

An absorbent article according to an embodiment of the invention has a total acquisition speed that is larger than 1 ml/s, preferably larger than 3 ml/s, most preferably larger than 3.4 ml/s.

According to the PACORM tests performed, the total acquisition speed of an absorbent article according to embodiments of the invention is 4 ml/s. The total acquisition speed is calculated using the acquisition speeds for all four gushes.

An absorbent article according to an embodiment of the invention has an acquisition speed after the first gush that is larger than 2 ml/s, preferably larger than 6 ml/s, most preferably larger than 7 ml/s.

According to the PACORM tests performed, the acquisition speed after the first gush, of an absorbent article according to embodiments of the invention is 8 ml/s.

The rewet value of the absorbent article is also tested with the method described in EP 0 797 967 B1. In EP 0 797 967 B1, the test method is referred to as "Comparative capillary rewet test", and the rewet value is defined as the weight increase of the blotting papers. Therein, blotting papers of a certain size are used. Herein the rewet value is defined as the weight increase in grams per square meter of blotting paper, such that the blotting papers used can be of any size.

An absorbent article according to an embodiment of the invention has a rewet value that is less than 13 g/m², preferably less than 11 g/m², most preferably less than 9 g/m².

According to tests performed, the rewet value of an absorbent article according to an embodiment of the invention is 8 g/m².

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. An absorbent article comprising:
a substantially fluid impervious backsheet (30);
a substantially fluid permeable topsheet (20);
an acquisition layer (40) for collecting and distributing fluid;
a top core (60) for absorbing fluid;
a bottom core (70) for absorbing fluid; and
elastic members (90);
the acquisition layer (40), the top core (60) and the bottom core (70) being arranged between the topsheet (20) and the backsheet (30), wherein the acquisition layer (40) is arranged between the topsheet (20) and the top core (60), and the bottom core (70) is arranged between the backsheet (30) and the top core (60),
wherein the absorbent article extends along a longitudinal axis from its front end (2) towards its rear end (3), the article having longitudinal side edges (4, 5) extending along the longitudinal axis, wherein the elastic members (90) are arranged adjacent to at least a portion of each longitudinal side edge (4, 5), each elastic member having a tension in a direction along the longitudinal side edge (4,5) ;
**characterized in that**
the top and the bottom cores (60, 70) comprise absorbent material that is compressed to a total density for the top and bottom core with a density of at least 0,13 g/cm³, preferably at least 0,15 g/cm³, most preferably at least 0,18 g/cm³, and wherein a distance (102) in the transversal direction between a side edge (4, 5) of the absorbent article (1) and a proximal side edge of the bottom core (70), in a position along the longitudinal direction where the bottom core has its smallest lateral extent, is 15-25 mm.

2. An absorbent article according to claim 1, wherein the bottom core (70) has a longitudinal extent (71) along the longitudinal axis, and wherein the top core (60) has a longitudinal extent (61) along the longitudinal axis that is 60-69 % of the longitudinal extent (71) of the bottom core (70), preferably 62-68 %, more preferably 64-68, most preferably 65-67 %.

3. An absorbent article according to any of claims 1 and 2, wherein an area of a body-facing surface of the top core (60) is 50-62 % of an area of a body-facing surface of the bottom core (70), preferably 52-61 %, more preferably 54-61 %, most preferably 55-60 %.

4. An absorbent article according to any of claims 1-3, wherein the elastic members (90) comprise foam elastics.

5. An absorbent article according to any of claims 1-3, wherein the elastic members (90) comprise elastic threads.

6. An absorbent article according to any of claims 1-3, wherein the elastic members (90) comprise an elastic film material.

7. An absorbent article according to any of claims 1-6, wherein each elastic member (90) is arranged to adopt
an initial relaxed length, when no external forces are acting on the elastic member, and
an outstretched length (95), when it is attached to the absorbent article and the absorbent article is in a flat outstretched condition, within a plane,
wherein the outstretched length is 140-180% of the initial relaxed length, preferably 150-170%, most preferably 155-165%.

8. An absorbent article according to any of claims 1-7, wherein each elastic member (90) is arranged such that it has an outstretched length (95) when it is attached to the absorbent article and the absorbent article is in a flat outstretched condition, within a plane, which outstretched length is about 40-61 % of the length of the bottom core (70), preferably 45-56 % most preferably 48-53 %.

9. An absorbent article according to any of claims 1-8, wherein the top core (60) and the bottom core (70) are hour glass shaped.

10. An absorbent article according to any of claims 1-9, wherein the top core (60) and the part of the bottom core (70) which is covered by the top core, have a retention value that corresponds to 60-90 %, preferably 70-80 % of a total retention value of the top core (60) and the bottom core (70).

11. An absorbent article according to any of claims 1-10, wherein the longitudinal extent of the bottom core (70) is 200-550 mm, preferably 350-500 mm, most preferably 445-460 mm.

12. An absorbent article according to any of claims 1-11, wherein a distance (101) in the transversal direction between an inner side edge (91) of the elastic member (90), which side edge faces towards the bottom core (70), and a proximal side edge of the bottom core (70), in a position along the longitudinal direction where the bottom core has its smallest lateral extent, is 4-12 mm, preferably 6-11 mm, most preferably 9-10 mm.

13. An absorbent article according to any of claims 1-12, wherein the distance (102) in the transversal direction between an side edge (4, 5) of the absorbent article (1) and a proximal side edge of the bottom core (70), in a position along the longitudinal direction where the bottom core has its smallest lateral extent, is 18-24 mm, or preferably 20-23 mm.

14. An absorbent article according to any of claim 1-13, wherein the total thickness of the compressed absorbent cores (60, 70) is less than 7 mm, preferably less than 6 mm.

15. An absorbent article according to any of claims 1-14 wherein the bottom core (70) has an embossed pattern 75.

## Patentansprüche

1. Saugfähiger Artikel, umfassend:
eine im wesentlichen flüssigkeitsundurchlässige Unterschicht (30);
eine im wesentlichen flüssigkeitsdurchlässige Oberschicht (20);
eine Aufnahmeschicht (40) zum Sammeln und Verteilen von Flüssigkeit;
einen oberen Kern (60) zum Absorbieren von Flüssigkeit;
einen Bodenkern (70) zum Absorbieren von Flüssigkeit; und
elastische Elemente (90);
wobei die Aufnahmeschicht (40), der obere Kern (60) und der untere Kern (70) zwischen der Oberschicht (20) und der Unterschicht (30) angeordnet sind, wobei die Aufnahmeschicht (40) zwischen der Oberschicht (20) angeordnet ist und der obere Kern (60) und der untere Kern (70) zwischen der Unterschicht (30) und dem oberen Kern (60) angeordnet sind,
wobei sich der saugfähige Artikel entlang einer Längsachse von seinem vorderen Ende (2) zu seinem hinteren Ende (3) erstreckt, wobei der Artikel Längsseitenkanten (4, 5) aufweist, die sich entlang der Längsachse erstrecken, wobei die elastischen Elemente (90) benachbart zu mindestens einem Abschnitt jeder Längsseitenkante (4, 5) angeordnet sind, wobei jedes elastische Element eine Spannung in einer Richtung entlang der Längsseitenkante (4, 5) aufweist;
**dadurch gekennzeichnet, dass**
der obere und der untere Kern (60, 70) saugfähiges Material umfassen, das auf eine Gesamtdichte für den oberen und den unteren Kern mit einer Dichte von mindestens 0,13 g/cm³, bevorzugt mindestens 0,15 g/cm³ komprimiert ist, am meisten bevorzugt mindestens 0,18 g/cm³, und wobei ein Abstand (102) in der Querrichtung zwischen einer Seitenkante (4, 5) des saugfähigen Artikels (1) und einer proximalen Seitenkante des unteren Kerns (70) in einer Position in Längsrichtung, in der der untere Kern die kleinste laterale Ausdehnung aufweist, 15-25 mm beträgt.

2. Saugfähiger Artikel nach Anspruch 1, wobei der untere Kern (70) eine Längserstreckung (71) entlang der Längsachse aufweist, und wobei der obere Kern (60) eine Längserstreckung (61) entlang der Längsachse aufweist, die 60 - 69 %, bevorzugt 62 - 68%, besonders bevorzugt 64 - 68 %, am meisten bevorzugt 65 - 67 % der Längserstreckung (71) des unteren Kerns (70) aufweist.

3. Saugfähiger Artikel nach einem der Ansprüche 1 und 2, wobei ein Bereich einer körperzugewandten Oberfläche des oberen Kerns (60) 50 - 62 % eines Bereichs einer körperzugewandten Oberfläche des unteren Kerns (70), bevorzugt 52 - 61%, besonders bevorzugt 54 - 61 %, am meisten bevorzugt 55 - 60 % beträgt.

4. Saugfähiger Artikel nach einem der Ansprüche 1 bis 3, wobei die elastischen Elemente (90) Schaumelastika umfassen.

5. Saugfähiger Artikel nach einem der Ansprüche 1 bis 3, wobei die elastischen Elemente (90) elastische Fäden umfassen.

6. Saugfähiger Artikel nach einem der Ansprüche 1 bis 3, wobei die elastischen Elemente (90) ein elastisches Filmmaterial umfassen.

7. Saugfähiger Artikel nach einem der Ansprüche 1 bis 6, wobei jedes elastische Element (90) so angeordnet ist, dass es
eine anfänglich entspannte Länge, wenn keine äußeren Kräfte auf das elastische Element einwirken, und
eine ausgestreckte Länge (95), wenn sie an dem saugfähigen Artikel befestigt ist und sich der saugfähige Artikel in einem flachen ausgestreckten Zustand innerhalb einer Ebene befindet,
annimmt,
wobei die ausgestreckte Länge 140 - 180 % der anfänglichen entspannten Länge, bevorzugt 150 - 170 %, am meisten bevorzugt 155 - 165 %, beträgt.

8. Saugfähiger Artikel nach einem der Ansprüche 1 bis 7, wobei jedes elastische Element (90) so angeordnet ist, dass es eine ausgestreckte Länge (95) aufweist, wenn es an dem saugfähigen Artikel angebracht ist, und sich der saugfähige Artikel in einem flach ausgestreckten Zustand innerhalb einer Ebene befindet, deren ausgestreckte Länge etwa 40 - 61 % der Länge des unteren Kerns (70), bevorzugt 45 - 56 %, am meisten bevorzugt 48 - 53 %, beträgt.

9. Saugfähiger Artikel nach einem der Ansprüche 1 bis 8, wobei der obere Kern (60) und der untere Kern (70) sanduhrförmig sind.

10. Saugfähiger Artikel nach einem der Ansprüche 1 bis 9, wobei der obere Kern (60) und der Teil des unteren Kerns (70), der vom oberen Kern bedeckt ist, einen Rückhaltewert aufweisen, der 60 - 90 %, bevorzugt 70 - 80 %, eines Gesamtretentionswertes des oberen Kerns (60) und des unteren Kerns (70) entspricht.

11. Saugfähiger Artikel nach einem der Ansprüche 1 bis 10, wobei die Längserstreckung des unteren Kerns (70) 200 bis 550 mm, bevorzugt 350 bis 500 mm, am meisten bevorzugt 445 bis 460 mm beträgt.

12. Saugfähiger Artikel nach einem der Ansprüche 1 bis 11, wobei ein Abstand (101) in Querrichtung zwischen einer inneren Seitenkante (91) des elastischen Elements (90), dessen Seitenkante dem Bodenkern (70) zugewandt ist, und einer proximalen Seitenkante des Bodenkerns (70) in einer Position entlang der Längsrichtung, in der der untere Kern seine kleinste laterale Ausdehnung hat, 4 - 12 mm, bevorzugt 6 - 11 mm, am meisten bevorzugt 9 - 10 mm beträgt.

13. Saugfähiger Artikel nach einem der Ansprüche 1 bis 12, wobei der Abstand (102) in Querrichtung zwischen einer Seitenkante (4, 5) des saugfähigen Artikels (1) und einer proximalen Seitenkante des Bodenkerns (70) in einer Position entlang der Längsrichtung, in welcher der Bodenkern seine kleinste laterale Ausdehnung aufweist, 18 - 24 mm oder bevorzugt 20 - 23 mm beträgt.

14. Saugfähiger Artikel nach einem der Ansprüche 1 bis 13, wobei die Gesamtdicke der komprimierten saugfähigen Kerne (60, 70) weniger als 7 mm, bevorzugt weniger als 6 mm beträgt.

15. Saugfähiger Artikel nach einem der Ansprüche 1 bis 14, wobei der Bodenkern (70) ein Prägemuster (75) aufweist.

## Revendications

1. Article absorbant, comprenant :
une feuille de support substantiellement imperméable au fluide (30) ;
une feuille supérieure substantiellement perméable au fluide (20) ;
une couche d'acquisition (40) destinée à collecter et à répartir du fluide ;
un noyau supérieur (60) destiné à absorber du fluide ;
un noyau inférieur (70) destiné à absorber du fluide ; et
des éléments élastiques (90) ;
la couche d'acquisition (40), le noyau supérieur (60) et le noyau inférieur (70) étant disposés entre la couche supérieure (20) et la feuille de support (30), la couche d'acquisition (40) étant disposée entre la feuille supérieure (20) et le noyau supérieur (60), et le noyau inférieur (70) étant disposé entre la feuille de support (30) et le noyau supérieur (60),
l'article absorbant s'étendant le long d'un axe longitudinal depuis son extrémité avant (2) vers son extrémité arrière (3), l'article ayant des bords latéraux longitudinaux (4, 5) s'étendant le long de l'axe longitudinal, les éléments élastiques (90) étant disposés adjacents à au moins une section de chaque bord latéral longitudinal (4, 5), chaque élément élastique présentant une tension dans un sens le long du bord latéral longitudinal (4, 5),
**caractérisé en ce que**
les noyaux supérieur et inférieur (60, 70) comprennent du matériau absorbant qui est compressé jusqu'à une densité totale pour le noyau supérieur et inférieur avec une densité d'au moins 0,13 g/cm³, de préférence au moins 0,15 g/cm³, le plus préférentiellement au moins 0,18 g/cm³, et une distance (102) dans le sens transversal entre un bord latéral (4, 5) de l'article absorbant (1) et un bord latéral proximal du noyau inférieur (70), à une position le long du sens longitudinal où le noyau inférieur a son extension latérale la plus réduite, est de 15 à 25 mm.

2. Article absorbant selon la revendication 1, dans lequel le noyau inférieur (70) a une extension longitudinale (71) le long de l'axe longitudinal, et le noyau supérieur (60) a une extension longitudinale (61) le long de l'axe longitudinal qui représente 60 à 69 % de l'extension longitudinale (71) du noyau inférieur (70), de préférence 62 à 68 %, plus préférentiellement 64 à 68, le plus préférentiellement 65 à 67 %.

3. Article absorbant selon l'une quelconque des revendications 1 et 2, dans lequel une zone d'une surface tournée vers le corps du noyau supérieur (60) représente 50 à 62 % d'une zone d'une surface tournée vers le corps du noyau inférieur (70), de préférence 52 à 61 %, plus préférentiellement 54 à 61 %, le plus préférentiellement 55 à 60 %.

4. Article absorbant selon l'une quelconque des revendications 1 à 3, dans lequel les éléments élastiques (90) comprennent de la mousse élastique.

5. Article absorbant selon l'une quelconque des revendications 1 à 3, dans lequel les éléments élastiques (90) comprennent des fils élastiques.

6. Article absorbant selon l'une quelconque des revendications 1 à 3, dans lequel les éléments élastiques (90) comprennent un film en matériau élastique.

7. Article absorbant selon l'une quelconque des revendications 1 à 6, dans lequel chaque élément élastique (90) est conçu pour adopter
une longueur relâchée initiale lorsqu'aucune force extérieure n'agit sur l'élément élastique, et
une longueur étirée (95) lorsqu'il est fixé à l'article absorbant et que l'article absorbant est dans une situation étirée à plat dans un plan,
la longueur étirée représentant 140 à 180 % de la longueur relâchée initiale, de préférence 150 à 170 %, le plus préférentiellement 155 à 165 %.

8. Article absorbant selon l'une quelconque des revendications 1 à 7, dans lequel chaque élément élastique (90) est disposé de manière à ce qu'il ait une longueur étirée (95) lorsqu'il est fixé à l'article absorbant et que l'article absorbant est dans une situation étirée à plat dans un plan, laquelle longueur étirée représente environ 40 à 61 % de la longueur du noyau inférieur (70), de préférence 45 à 56 %, le plus préférentiellement 48 à 53 %.

9. Article absorbant selon l'une quelconque des revendications 1 à 8, dans lequel le noyau supérieur (60) et le noyau inférieur (70) ont une forme de verre de montre.

10. Article absorbant selon l'une quelconque des revendications 1 à 9, dans lequel le noyau supérieur (60) et la partie du noyau inférieur (70) qui est recouverte par le noyau supérieur ont une valeur de rétention qui correspond à 60 à 90 %, de préférence 70 à 80 %, d'une valeur de rétention totale du noyau supérieur (60) et du noyau inférieur (70).

11. Article absorbant selon l'une quelconque des revendications 1 à 10, dans lequel l'extension longitudinale du noyau inférieur (70) est de 200 à 550 mm, de préférence 350 à 500 mm, le plus préférentiellement 445 à 460 mm.

12. Article absorbant selon l'une quelconque des revendications 1 à 11, dans lequel une distance (101) dans le sens transversal entre un bord latéral intérieur (91) de l'élément élastique (90), lequel bord latéral est tourné vers le noyau inférieur (70), et un bord latéral proximal du noyau inférieur (70), à une position dans le sens longitudinal où le noyau inférieur à son extension latérale la plus réduite, est de 4 à 12 mm, de préférence 6 à 11 mm, le plus préférentiellement 9 à 10 mm.

13. Article absorbant selon l'une quelconque des revendications 1 à 12, dans lequel la distance (102) dans le sens transversal entre un bord latéral (4, 5) de l'article absorbant (1) et un bord latéral proximal du noyau inférieur (70), à une position le long du sens longitudinal où le noyau inférieur a son extension latérale la plus réduite, est de 18 à 24 mm, de préférence 20 à 23 mm.

14. Article absorbant selon l'une quelconque des revendications 1 à 13, dans lequel l'épaisseur totale des noyaux absorbants compressés (60, 70) est inférieure à 7 mm, de préférence inférieure à 6 mm.

15. Article absorbant selon l'une quelconque des revendications 1 à 14, dans lequel le noyau inférieur (70) comporte un motif gaufré (75).
